(19) Europäisches Patentamt
European Patent Office
Office européen des brevets

(11) Numéro de publication: **0 037 353**
**B1**

(12) # FASCICULE DE BREVET EUROPEEN

(45) Date de publication du fascicule du brevet: **08.06.83**

(51) Int. Cl.³: **C 07 C 41/09,** C 07 C 43/23, C 07 C 43/205

(21) Numéro de dépôt: **81420047.3**

(22) Date de dépôt: **30.03.81**

(54) **Procédé d'éthérification de phénols.**

(30) Priorité: **31.03.80 FR 8007628**

(43) Date de publication de la demande:
**07.10.81 Bulletin 81/40**

(45) Mention de la délivrance du brevet:
**08.06.83 Bulletin 83/23**

(84) Etats contractants désignés:
**BE CH DE FR GB IT LI NL**

(56) *Documents cités:*
**EP-A-0 000 162**
**FR-A-2 255 279**

**BULLETIN OF THE CHEMICAL SOCIETY OF JAPAN, vol. 45, 1972 T. KITO et al.: "O-Alkylation of phenols by esters", *pages 3490-3492***

(73) Titulaire: **RHONE-POULENC SPECIALITES CHIMIQUES, "Les Miroirs" 18, Avenue d'Alsace, F-92400 Courbevoie (FR)**

(72) Inventeur: **Ratton, Serge, Vaulx en Milieu, F-38290 La Verpillière (FR)**

(74) Mandataire: **Vignally, Noel et al, RHONE-POULENC RECHERCHES Centre de recherches de Saint Fons Service Brevets Boîte postale 62, F-69190 Saint Fons (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 037 353 B1

# Procédé d'éthérification de phénols

La présente invention concerne un nouveau procédé d'éthérification sélective d'une fonction phénolique de composés pouvant en comporter plusieurs.

Il est connu d'éthérifier les fonctions phénoliques à l'aide d'un sulfate d'alkyle ou d'un halogénure d'alkyle [Houben-Weyl Methoden der Organischen Chemie, Vol. III, page 54 (1965)].

Cette méthode peut permettre d'obtenir sélectivement un monoéther lorsque l'on utilise des quantités stœchiométriques de réactifs. Mais les réactifs mis en œuvre sont chers et, en outre, la réaction donne naissance à des sels minéraux posant des problèmes de corrosion des appareillages et de toxicité des effluents.

Récemment la demande de brevet japonais n° 79/030123 a montré que l'on peut éthérifier les fonctions phénoliques de composés aromatiques dihydroxylés, par l'action de phosphates d'alkyle; mais il n'est pas possible d'empêcher une réaction importante de diéthérification.

La demande de brevet français n° 74-18172 (publiée sous le numéro 2231649) décrit un procédé d'éthérification de composés phénoliques, ayant un ou plusieurs groupements hydroxyle, par un alcool aliphatique saturé ayant 1 à 4 atomes de carbone ou un ester d'un tel alcool et d'un acide aliphatique saturé carboxylique, en présence d'une amine tertiaire aliphatique ou d'un chlorure, d'un sulfate ou d'un carboxylate d'une telle amine. Cependant il s'avère que, lorsque ce procédé est appliqué à un composé phénolique dihydroxylé, on obtient un mélange de mono- et de diéthers.

Enfin la demande de brevet français n° 77-37263 (publiée sous le n° 2373506) décrit un procédé de préparation d'oxydes d'alkyle et d'aryle par réaction, en présence de résines échangeuses de cations fortement acides, d'un composé aromatique hydroxylé avec un alcool aliphatique, ce procédé se caractérisant en ce que l'on opère avec au moins 3 moles de composé aromatique hydroxylé par mole d'alcool aliphatique. La température de mise en œuvre du procédé est située de préférence entre 110°C et 130°C. Ce procédé permet d'obtenir sélectivement le monoéther à partir d'un polyphénol. Le principal inconvénient d'un tel procédé réside dans la cherté des résines utilisées comme catalyseur et dans leur sensibilité à la chaleur qui risque de provoquer leur dégradation.

Le «Bulletin of the Chemical Society of Japan», volume 45/1972 (pages 3490 à 3492) décrit un procédé d'O-alkylation de monophénols, éventuellement substitués, dont la fonction phénolique est sous forme de phénolate de sodium ou de potassium. Un tel procédé n'est pas présenté comme étant applicable à la monoéthérification sélective des polyphénols.

La demande de brevet européen publiée sous le n° 00162 préconise l'O-alkylation de mono- ou de diphénols à l'aide de carbonate de dialkyle ou de diaryle en présence d'une amine ou d'une phosphine tertiaires. Ce procédé permet l'obtention sélective de monoéthers, mais il met en œuvre des composés relativement coûteux et pose des problèmes délicats de traitemnt des essais et de recyclage des catalyseurs.

La demande de brevet français n° 74.38803 (publiée sous le n° 2255279) décrit un procédé de monoéthérification de polyphénols en milieu solvant neutre dipolaire comportant un groupement sulfoxyde, sulfone ou amide, en présence d'un oxyde ou d'un hydroxyde de métal alcalino-terreux. Les solvants utilisés sont coûteux et le procédé pose en outre des problèmes de traitement de la masse réactionnelle finale et des effluents produits.

Il a maintenant été trouvé un nouveau procédé, constituant la présente invention, qui met en jeu des réactifs peu coûteux et qui conduit à l'éthérification sélective d'une fonction phénolique d'un composé pouvant en comporter plusieurs.

L'éthérification est dite sélective parce qu'au cours de la mise en œuvre du procédé selon l'invention, une seule fonction phénolique est éthérifiée, lorsque ledit composé phénolique en comporte plusieurs sur le même cycle aromatique.

Plus précisément, l'invention a pour objet un nouveau procédé d'éthérification d'une fonction phénolique d'un composé de formule générale:

$$HO\text{-}Ar\text{-}(R)_n \qquad (I)$$

dans laquelle:

- Ar représente un radical aromatique constitué par un cycle benzénique ou par une structure formée par plusieurs cycles benzéniques orthocondensés ou ortho- et péricondensés,
- les substituants R, identiques ou différents, représentent un groupement hydroxyle, un radical alkyle linéaire ou ramifié ayant de 1 à 6 atomes de carbone, un radical alkényle linéaire ou ramifié ayant 2 à 6 atomes de carbone, un radical phényle éventuellement substitué, un radical cycloalkyle éventuellement substitué, un radical phénylalkyle dans lequel la chaîne aliphatique comporte 1 à 4 atomes de carbone, un radical cycloalkylalkyle dans lequel la chaîne aliphatique comporte de 1 à 4 atomes de carbone, un atome d'halogène, un groupement nitro, un groupement amine, un groupement aldéhyde -CHO, ou un groupement nitrile,
- n est un nombre de 0 à 5,

par réaction avec un agent d'éthérification choisi dans le groupe constitué par les carboxylates d'alkyle, le radical alkyle linéaire ou ramifié ayant 1 à 6 atomes de carbone, les carboxylates d'alkényle, le radical alkényle linéaire ou ramifié ayant 3 à 6 atomes de carbone et les composés susceptibles de former de tels carboxylates, caractérisé en ce que l'on opère en présence d'un sel d'acide carboxylique choisi parmi les carboxylates de métal alcalin, d'ammonium ou de métal alcalino-terreux.

L'invention peut également être présentée comme un procédé de préparation de monoéther de phénol par réaction d'un composé de formule générale (I) avec un agent d'éthérification choisi dans le groupe constitué par les carboxylates d'alkyle, le radical alkyle linéaire ou ramifié ayant 1 à 6 atomes de carbone, les carboxylates d'alkényle, le radical alkényle linéaire ou ramifié ayant 3 à 6 atomes de carbone et les composés susceptibles de former de tels carboxylates, ledit procédé se caractérisant en ce que l'on opère en présence d'un sel d'acide carboxylique parmi ceux indiqués ci-dessus.

De manière préférée, le procédé peut être appliqué à des composés phénoliques de formule (I) dans laquelle:

- le radical Ar correspond au benzène, au naphtalène, à l'anthracène ou au phénanthrène,
- les substituants R, identiques ou différents, représentent un groupement hydroxyle, un radical alkyle linéaire ou ramifié ayant 1 à 4 atomes de carbone tel que méthyle, éthyle, isopropyle, n-propyle, n-butyle, isobutyle ou tertiobutyle, un radical alkényle linéaire ou ramifié ayant 2 à 4 atomes de carbone, tel que vinyle, allyle, propène-1 yle, isopropényle, butène-1 yle, butène-2 yle, butène-3 yle, méthyl-1 propène-1 yle, méthyl-1 propène-2 yle, méthyl-2 propène-1 yle, méthyl-2 propène-2 yle, un radical phényle éventuellement substitué, un radical cyclohexyle éventuellement substitué, un radical phénylalkyle dans lequel la chaîne aliphatique comporte de 1 à 3 atomes de carbone tel que benzyle, phénéthyle, phénylpropyle et phénylisopropyle, un radical cyclohexylalkyle dans lequel la chaîne aliphatique comporte 1 à 3 atomes de carbone, un atome de chlore, un atome de brome ou un groupement nitro,
- n est un nombre entier de 0 à 3.

A titre d'exemples de tels composés phénoliques, on peut citer:
- les monophénols comme le phénol, le naphtol-1, le naphtol-2, le phénanthrol-1, le phénanthrol-2, le phénanthrol-3, le phénanthrol-9, l'anthrol-1, l'anthrol-2, l'anthrol-9
- les diphénols comme le résorcinol, la pyrocatéchol, l'hydroquinone, le dihydroxy-1,2 naphtalène, le dihydroxy-1,3 naphtalène, le dihydroxy-1,4-naphtalène, le dihydroxy-1,5 naphtalène, le dihydroxy-1,6 naphtalène, le dihydroxy-1,7 naphtalène, le dihydroxy-1,8 naphtalène, le dihydroxy-2,3 naphtalène, le dihydroxy-2,6 naphtalène, le dihydroxy-2,7 naphtalène, le dihydroxy-1,2 anthracène, le dihydroxy-1,5 anthracène, le dihydroxy-1,8 anthracène, le dihydroxy-2,6 anthracène, le dihydroxy-9,10 anthracène, le dihydroxy-3,4 phénanthrène,
- les triphénols comme le pyrogallol, le trihydroxy-1,2,4 benzène, le trihydroxy-1,3,5 benzène, le trihydroxy-1,2,9 anthracène, le trihydroxy-1,2,10 anthracène, le trihydroxy-1,4,9 anthracène, le trihydroxy-1,5,9 anthracène, le trihydroxy-1,8,9 anthracène, le trihydroxy-2,3,9 anthracène, le trihydroxy-3,4,5 phénanthrène,

les mono- ou diphénols portant également 1 ou plusieurs autres substituants R comme: le chloro-2 phénol, le chloro-3 phénol, le chloro-4 phénol, le bromo-2 phénol, le bromo-3 phénol, le bromo-4 phénol, le nitro-2 phénol, le nitro-3 phénol, le nitro-4 phénol, le méthyl-2 phénol, le méthyl-3 phénol, le méthyl-4 phénol, l'éthyl-2 phénol, l'éthyl-3 phénol, l'éthyl-4 phénol, l'isopropyl-2 phénol, l'isopropyl-3 phénol, l'isopropyl-4 phénol, le propyl-2 phénol, le propyl-3 phénol, le propyl-4 phénol, le (propène-1 yle)-4 phénol, l'allyl-2 phénol, l'allyl-4 phénol, le butyl-3 phénol, le butyl-4 phénol, l'isobutyl-4 phénol, le tertiobutyl-2 phénol, le tertiobutyl-3 phénol, le tertiobutyl-4 phénol, le benzyl-2 phénol, le benzyl-4 phénol, le cyclohexyl-2 phénol, le dichloro-2,3 phénol, le dichloro-2,4 phénol, le dichloro-2,5 phénol, le dichloro-2,6 phénol, le dichloro-3,4 phénol, le dichloro-3,5 phénol, le diméthyl-1,2 hydroxy-3 benzène, le diméthyl-1,2 hydroxy-4 benzène, le diméthyl-1,3 hydroxy-5 benzène, le diméthyl-1,3 hydroxy-2 benzène, le diméthyl-1,4 hydroxy-2 benzène, le diméthyl-2,4 hydroxy-1 benzène, le tertiobutyl-1 hydroxy-2 méthyl-4 benzène, le tertiobutyl-2 hydroxy-1 méthyl-4 benzène, le tertiobutyl-2 éthyl-4 hydroxy-1 benzène, le tertiobutyl-4 éthyl-2 hydroxy-1 benzène, le ditertiobutyl-1,3 hydroxy-2 benzène, le ditertiobutyl-2,4 hydroxy-1 benzène, l'hydroxy-2 isopropyl-4 méthyl-1 benzène, l'allyl-2 chloro-4 hydroxy-1 benzène, l'hydroxy-1 triméthyl-2,4,5 benzène, l'hydroxy-2 triméthyl-1,3,5 benzène, l'hydroxy-2 tritertiobutyl-1,3,5 benzène, le ditertiobutyl-1,3 hydroxy-2 méthyl-5 benzène, le ditertiobutyl-1,5 hydroxy-2 méthyl-3 benzène, le ditertiobutyl-1,5 hydroxy-2 méthyl-4 benzène, le tertiobutyl-1 diméthyl-2,5 hydroxy-4 benzène, le tertiobutyl-1 diméthyl-3,5 hydroxy-2 benzène, le tertiobutyl-1 diméthyl-4,5 hydroxy-2 benzène, le tertiboutyl-5 diméthyl-1,3 hydroxy-2 benzène, le chloro-1 diméthyl-2,3 hydroxy-4 benzène, le chloro-1 diméthyl-2,3 hydroxy-5 benzène, le chloro-1 diméthyl-2,4 hydroxy-5 benzène, le chloro-1 diméthyl-2,5 hydroxy-4 benzène, le chloro-1 diméthyl-3,4 hydroxy-2 benzène, le chloro-1 diméthyl-4,5 hydroxy-2 benzène, le chloro-2 diméthyl-1,3 hydroxy-5 benzène, le chloro-2 diméthyl-1,5 hydroxy-3 benzène, le chloro-2 diméthyl-3,4 hydroxy-1 benzène, le chloro-5 diméthyl-1,3 hydroxy-2 benzène, le diméthyl-1,2 hydroxy-3 nitro-5 benzène, le diméthyl-1,2 hydroxy-4 nitro-5 benzène, le diméthyl-1,3 hydroxy-2 nitro-4 benzène, le diméthyl-1,3 hydroxy-2 nitro-5 benzène, le diméthyl-1,4 hydroxy-2 nitro-3 benzène, le diméthyl-1,4 hydroxy-2 nitro-5 benzène, le diméthyl-1,5 hydroxy-2 nitro-3 benzène, le diméthyl-1,5 hydroxy-3 nitro-2 benzène, le diméthyl-2,5 hydroxy-1 nitro-3 benzène, le nitro-8 naphtol-1, le nitro-1 naphtol-2, le nitro-5 naphtol-2, le méthyl-1 naphtol-2, le bromo-1 dihydroxy-2,4 benzène, le bromo-1 dihydroxy-3,5 benzène, le bromo-2 dihydroxy-1,3 benzène, le bromo-2 dihydroxy-1,4 benzène, le bromo-4 dihydroxy-1,2 benzène, le

butyl-1 dihydroxy-2,4 benzène, le chloro-1 dihydroxy-2,3 benzène, le chloro-1 dihydroxy-2,4 benzène, le chloro-1 dihydroxy-3,5 benzène, le chloro-2 dihydroxy-1,3 benzène, le chloro-2 dihydroxy-1,4 benzène, le chloro-4 dihydroxy-1,2 benzène, le dihydroxy-2,4 éthyl-1 benzène, le dihydroxy-2,4 isobutyl-1 benzène, le dihydroxy-1,2 isopropyl-4 benzène, le dihydroxy-1,4 isopropyl-2 benzène, le dihydroxy-2,4 isopropyl-1 benzène, le dihydroxy-2,3 isopropyl-1 méthyl-4 benzène, le dihydroxy-1,4 isopropyl-2 méthyl-5 benzène, le dihydroxy-1,2 méthyl-3 benzène, le dihydroxy-1,3 méthyl-2 benzène, le dihydroxy-1,3 nitro-2 benzène, le dihydroxy-1,4 nitro-2 benzène, le dihydroxy-1,2 propyl-4 benzène, le dihydroxy-1,3 propyl-5 benzène, le dihydroxy-2,4 propyl-1 benzène, le dichloro-1,2 dihydroxy-4,5 benzène, le dichloro-1,3 dihydroxy-2,5 benzène, le dichloro-1,4 dihydroxy-2,5 benzène, le dichloro-1,5 dihydroxy-2,3 benzène, le dichloro-1,5 dihydroxy-2,4 benzène, le dichloro-2,3 dihydroxy-1,4 benzène, le dihydroxy-1,2 diméthyl-3,5 benzène, le dihydroxy-1,2 diméthyl-4,5 benzène, le dihydroxy-1,3 diméthyl-2,4 benzène, le dihydroxy-1,3 diméthyl-2,5 benzène, le dihydroxy-1,4 diméthyl-2,3 benzène, le dihydroxy-1,4 diméthyl-2,5 benzène, le dihydroxy-1,5 diméthyl-2,4 benzène, le dihydroxy-1,5 diméthyl-3,4 benzène, le dihydroxy-2,5 diméthyl-1,3 benzène, le dihydroxy-1,3 dinitro-2,4 benzène.

Le procédé selon l'invention est plus particulièrement appliqué au phénol, au naphtol-1, au naphtol-2, au méthyl-2 phénol., au méthyl-3 phénol, au méthyl-4 phénol, aux monochlorophénols, aux dichlorophénols, aux monoéthylphénols, au pyrocatéchol, au résorcinol, à l'hydroquinone, au dihydroxy-1,2 naphtalène, au dihydroxy-1,3 naphtalène, au dihydroxy-1,4 naphtalène, au dihydroxy-1,5 naphtalène, au dihydroxy-1,6 naphtalène, au dihydroxy-1,7 naphtalène, au dihydroxy-1,8 naphtalène, au dihydroxy-2,3 naphtalène, au dihydroxy-2,6 naphtalène, au dihydroxy-2,7 naphtalène.

La concentration du composé phénolique de formule (I) dans le milieu réactionnel n'est pas critique. Elle varie très largement, notamment en fonction de la solubilité de ce composé dans ledit milieu, qui est constitué par l'agent d'éthérification, le sel d'acide carboxylique et le cas échéant par d'autres éléments qui seront détaillés plus loin et qui peuvent consister en particulier en des adjuvants non indispensables, mais favorables à la mise en œuvre du procédé selon l'invention et/ou en un tiers solvant.

Pour des raisons de commodité, la concentration du composé phénolique sera exprimée par rapport au milieu liquide, c'est-à-dire au milieu réactionnel dont on exclut le composé phénolique lui-même et le sel d'acide carboxylique.

C'est ainsi que généralement on opère avec de 1% à 100% en poids de composé phénolique par rapport au volume du milieu liquide. Le plus fréquemment cette concentration est comprise entre 2% et 80% en poids par volume.

Parmi les agents d'éthérification que l'on peut mettre en œuvre dans le procédé selon l'invention, on peut citer notamment les esters:

- des acides carboxyliques aliphatiques saturés ou insaturés monofonctionnels comportant notamment 2 à 18 atomes de carbone ou polyfonctionnels comportant notamment 3 à 18 atomes de carbone,
- des acides aromatiques mono- ou polyfonctionnels,
- des acides arylaliphatiques mono- ou polyfonctionnels,
- ou des acides cycloaliphatiques mono- ou polyfonctionnels avec des alcools tels que le méthanol, l'éthanol, le n-propanol, l'isopropanol, le butanol-1, le butanol-2, le tertiobutanol, le propène-2 ol-1, le méthyl-2 propène-2 ol-1, le butène-2 ol-1, le butène-3 ol-1, le butène-3 ol-2.

On peut également mettre en œuvre des composés susceptibles de former de tels esters in situ et notamment un mélange d'un alcool tel que ceux énumérés ci-avant avec un acide carboxylique tel que ceux définis précédemment. Cette variante est celle qui est préférée notamment pour des raisons de commodité, ces réactifs étant parfois plus facilement disponibles que les esters eux-mêmes. En outre, il n'est pas nécessaire d'avoir des quantités stœchiométriques d'alcool et d'acide carboxylique. Enfin la réaction d'éthérification est en général plus rapide avec ces réactifs qu'avec les esters. Le rapport molaire alcool/acide carboxylique peut varier dans de larges limites, par exemple entre 0,02 et 50, plus fréquemment entre 0,1 et 40. Le rapport molaire acide carboxylique/composé phénolique peut également varier très largement. Généralement il se situe entre 0,1 et 100. De préférence ce rapport est compris entre 0,5 et 50.

Par la suite lorsqu'il sera question d'agent d'éthérification, ce terme englobera aussi bien l'ester lui-même que les mélanges dans les proportions définies précédemment de l'alcool et de l'acide carboxylique correspondants.

La quantité d'agent d'éthérification utilisée est généralement choisie de telle façon que le rapport molaire ester et/ou alcool entrant dans la composition dudit agent éthérifiant sur composé phénolique de formule (I) est égal ou supérieur à 0,5 et de préférence égal ou supérieur à 1.

On peut citer à titre d'illustration des acides carboxyliques précédents des monoacides aliphatiques saturés comme l'acide acétique, le propanoïque, le n-butanoïque, le méthyl-2 propanoïque, le n-pentanoïque, le méthyl-2 butanoïque, le méthyl-3 butanoïque, le diméthyl-3,3 butanoïque, le n-hexanoïque, le méthyl-2 pentanoïque, le méthyl-3 pentanoïque, le méthyl-4 pentanoïque; des monoacides aliphatiques insaturés comme le propènoïque, le butène-2 oïque, le méthyl-2 propènoïque, le butène-3 oïque, le méthyl-2 butène-2 oïque cis (acide angélique), le méthyl-2 butène-2 oïque trans (acide tiglique), le pentène-4 oïque, l'hexène-3 oïque, l'hexène-4 oïque; des diacides aliphatiques saturés comme l'acide malonique, l'acide succinique, l'acide glutarique, l'acide adipique; des diacides éthyléniques comme l'acide maléïque et l'acide fumarique; des mono- ou des diacides aromatiques comme l'acide benzoïque, l'acide orthophtalique, l'acide isophtalique, l'acide téréphtalique, les acides mononitrobenzoïques, les acides monochlorobenzoïques; des acides arylaliphatiques comme l'acide phénylacétique, le phényl-2 propanoïque, le phényl-4 propanoïque; des acides cycloaliphatiques comme l'acide cyclohexanedicarboxylique-1,3, l'acide cyclohexanedicarboxylique-1,4.

On peut également utiliser des mélanges comprenant un ester d'acide carboxylique et l'alcool libre correspondant ou un ester d'acide carboxylique avec l'alcool libre et l'acide carboxylique libre correspondant à cet ester. Dans le présent texte lorsque l'on parle d'agent d'étherification, il est entendu qu'il peut s'agir d'un tel mélange, sans qu'il soit nécessaire de le préciser chaque fois.

Les agents d'éthérification que l'on préfère utiliser généralement sont les esters du méthanol, de l'éthanol, du n-propanol, du propène-2 ol-1 et du méthyl-2 propène-2 ol-1 et d'un acide carboxylique, ou les mélanges tels que définis précédemment de l'un de ces alcools avec un acide carboxylique. On choisit fréquemment l'acide carboxylique parmi les acides aliphatiques saturés mono- ou difonctionnels ayant 2 à 6 atomes de carbone, l'acide benzoïque et les acides ortho-, méta- ou téréphtaliques. Parmi ces agents d'éthérification, on préfère le plus souvent utiliser les acétates, les propionates et les succinates ou les mélanges constitués par l'acide acétique oul'acide propionique ou l'acide succinique avec l'un des alcools cités ci-avant.

Enfin parmi ces agents d'éthérification préférés, on préfère plus particulièrement employer les esters du méthanol et les esters de l'éthanol, tout spécialement leurs acétates ou les mélanges constitués par le méthanol et l'un de ces acides carboxyliques ou l'éthanol et l'un de ces acides carboxyliques et tout spécialement les mélanges constitués par l'un de ces deux alcools avec l'acide acétique.

L'agent d'éthérification peut constituer le milieu solvant dans lequel s'opère la réaction d'éthérification selon le procédé de l'invention. Mais il est bien entendu que l'on peut également utiliser un tiers solvant, liquide dans les conditions de mise en œuvre du procédé, dans la mesure où ledit tiers solvant est inerte vis-à-vis des réactifs et stable aux températures auxquelles est effectuée la réaction.

L'eau peut servir de tiers solvant. Mais sa présence procure un avantage particulier et joue un rôle remarquable, distinct du simple rôle de tiers solvant. En effet la présence d'eau conduit à une augmentation du rendement en monoéther du composé phénolique et à une diminution corrélative des réactions parasites.

Lorsque l'on opère en présence d'eau, celle-ci peut représenter de 1% à 95% en volume du milieu réactionnel liquide. De préférence le milieu réactionnel liquide comprend de 20% à 80% en volume d'eau.

Le sel d'acide carboxylique servant de catalyseur dans le procédé selon l'invention peut être tout carboxylate des métaux alcalins, d'ammonium et des métaux alcalino-terreux. A titre d'exemples, on peut citer notamment les carboxylates de sodium, de potassium, de lithium et d'ammonium; on peut également citer les carboxylates de calcium, de magnésium et de baryum.

Les acides carboxyliques servant à obtenir de tels carboxylates peuvent être les acides mono- ou polyfonctionnels, aliphatiques saturés ou insaturés, aromatiques, arylaliphatiques, cycloaliphatiques dont les cycles peuvent être substitués par un ou plusieurs radicaux.

Ces acides peuvent être par exemple ceux qui ont été cités précédemment lors de la définition des esters servant d'agents d'éthérification.

Parmi tous les carboxylates que l'on peut utiliser on préfère généralement les sels des acides carboxyliques aliphatiques saturés, monofonctionnels ayant de 2 à 6 atomes de carbone, tels que notamment l'acide acétique, le propanoïque, le n-butanoïque, le n-pentanoïque, le n-hexanoïque, le méthyl-2 propanoïque, le méthyl-2 butanoïque, le méthyl-3 butanoïque, le diméthyl-3,3 butanoïque, le méthyl-2 pentanoïque, le méthyl-3 pentanoïque et le méthyl-4 pentanoïque, ou difonctionnels ayant 3 à 6 atomes de carbone, tels que notamment l'acide malonique, l'acide succinique, l'acide glutarique et l'acide adipique, les sels de l'acide benzoïque et ceux des acides orthophtalique, isophtalique et téréphtalique. Parmi les sels de ces acides on préfère les sels de métaux alcalins.

De manière encore préférée, on emploie les sels de sodium ou de potassium de ces acides. Parmi ces derniers sels, on utilise préférentiellement l'acétate de sodium, le propionate de sodium et le succinate de sodium.

Il est commode de choisir l'agent d'éthérification et le sel d'acide carboxylique servant de catalyseur de telle façon qu'ils proviennent (ou contiennent) du même acide carboxylique, mais cela n'est pas indispensable.

La quantité de sel d'acide carboxylique présent dans le milieu peut varier dans de larges limites. Si on exprime cette quantité par rapport au composé phénolique, elle n'est généralement pas inférieure à 0,05 fois le poids dudit composé phénolique. La quantité maximale n'est pas critique. Habituellement elle ne dépasse pas 50 fois le poids du composé phénolique. On préfère le plus souvent utiliser des rapports pondéraux sel d'acide carboxylique/composé phénolique variant de 0,1 à 20.

Une variante intéressante consiste à mettre en œuvre un diacide carboxylique dont une fonction acide est salifiée, en général par un métal alcalin, et dont l'autre fonction est soit libre, soit estérifiée par un alcool tel que ceux précédemment cités, le plus souvent le méthanol ou l'éthanol.

Sur un plan pratique, afin d'obtenir un très bon

rendement en monoéther par rapport au composé phénolique transformé, tout en ayant une mise en œuvre et un traitement final du mélange réactionnel relativement faciles, on choisit de préférence la réalisation suivante du procédé selon l'invention. On opère généralement dans un milieu constitué par le mélange alcool + acide carboxylique qui joue le double rôle de réactif et de solvant; on ajoute également de l'eau, à raison le plus souvent d'un volume égal à celui de l'alcool et un carboxylate de métal alcalin, notamment de sodium, dérivant de l'acide carboxylique libre utilisé.

Les quantités relatives des divers réactifs ou constituants du mélange réactionnel sont alors choisies dans les zones préférentielles indiquées précédemment.

Le procédé selon l'invention nécessite pour sa mise en œuvre un chauffage des réactifs; la température à laquelle on opère peut varier de 150°C à 350°C. Elle se situe de préférence entre 220°C et 300°C.

La pression n'est pas un paramètre critique de la réaction. Habituellement elle est constituée par la pression autogène obtenue par chauffage, à la température désirée, du mélange réactionnel dans un appareillage adéquat fermé. Elle se situe généralement entre 10 bars et 100 bars. Mais elle peut atteindre des valeurs plus élevées car il est possible, sans sortir du cadre de l'invention, de créer dans l'appareil utilisé pour la réaction, une pression initiale à froid supérieure à la pression atmosphérique, par exemple au moyen d'un gaz inerte tel que l'azote.

L'appareillage utilisé n'est pas spécifique au procédé de l'invention. Simplement il doit présenter certaines caractéristiques: il doit pouvoir résister aux pressions que l'on atteint lors du chauffage, il doit être étanche et évidemment ne pas être attaqué par les réactifs utilisés.

Pratiquement le procédé selon l'invention peut être mis en œuvre de la manière suivante: on charge les différents constituants du mélange réactionnel, tels que définis précédemment, dans l'appareillage adéquat. On chauffe à la température désirée, de préférence sous agitation mais sans que celà soit véritablement indispensable, pendant une durée pouvant varier de quelques minutes à plus de 20 heures par exemple. Cependant cette durée est généralement de l'ordre de quelques heures, par exemple de 2 heures à 10 heures selon la température à laquelle on opère.

En fin de réaction, l'appareil est refroidi et la masse réactionnelle finale est traitée de manière classique, selon les réactifs utilisés; si le milieu contient de l'eau, les composés organiques autres que le sel d'acide carboxylique sont extraits par un solvant non miscible à l'eau. Si le milieu ne contient pas ou très peu d'eau, on peut généralement séparer le sel d'acide carboxylique par filtration, soit directement, soit après l'avoir précipité par addition d'un solvant organique dans lequel il n'est pas soluble, mais qui dissout les composés formés lors de la réaction. On peut aussi rajouter de l'eau au milieu avant de procéder à l'extraction des composés organiques.

Les produtis obtenus sont séparés, notamment du composé phénolique n'ayant pas été transformé, par des opérations courantes dans le domaine de la chimie, puis dosés si nécessaire, également par des méthodes bien connues de l'homme du métier.

Un procédé de séparation des éthers formés au cours de la réaction consiste notamment à les extraire à l'aide d'un solvant non miscible à l'eau approprié. On peut utiliser par exemple le cyclohexane comme solvant d'extraction. De préférence on opère à une température comprise entre 50°C et le point d'ébullition du mélange réactionnel final. En général cette température, non critique, est de l'ordre de 60°C. On extrait ainsi la grande majorité des produits d'éthérification formés, tandis que le phénol non transformé reste pour l'essentiel dans le milieu réactionnel avec le sel d'acide carboxylique et l'acide carboxylique libre. La masse réactionnelle finale obtenue après l'extraction peut ainsi être recyclée après un éventuel rajout d'agent d'éthérification.

Les monoéthers obtenus par le procédé selon l'invention peuvent soit être utilisés directement, soit servir d'intermédiaires pour la synthèse de composés plus complexes.

Le méthoxy-2 phénol (ou gaïacol) par exemple est utilisé dans l'industrie pharmaceutique; il sert également d'intermédiaire pour la préparation de la vanilline.

Dans les exemples qui vont suivre les dosages, sauf mention contraire, sont effectués par chromatographie gaz/liquide.

Exemple 1

Dans un tube de verre résistant à la pression, on introduit les réactifs suivants:

| | |
|---|---|
| - Acétate de sodium anhydre | 9,18 g |
| - Acide acétique | 0,75 g |
| - Eau distillée | 5 ml |
| - Méthanol | 5 ml |
| - Pyrocatéchol | 0,51 g |

Le tube est scellé, puis on chauffe à 250°C sous agitation et on maintient à cette température pendant 5 heures.

En fin d'essai, on refroidit et on extrait du mélange aqueux le pyrocatéchol non transformé et le gaïacol formé, à l'aide d'éther isopropylique. On dose les produits par chromatographie gaz/liquide. On trouve:

- Pyrocatéchol non transformé: 0,344 g, soit un taux de transformation (TT) du pyrocatéchol de 32,5%.
- Gaïacol formé: 0,190 g, soit un rendement par rapport au pyrocatéchol transformé (RT) de 100%.

Exemple 2

Dans un tube de verre résistant à la pression, on introduit les réactifs suivants:

| | |
|---|---|
| - Acétate de sodium anhydre | 2,3 g |
| - Acide acétique | 0,38 g |
| - Eau distillée | 5 ml |
| - Méthanol | 5 ml |
| - Pyrocatéchol | 0,501 g |

Le tube est scellé, puis chauffé sous agitation à 250°C et maintenu 5 heures à cette température.

Le mélange réactionnel final est traité et dosé comme dans l'exemple 1:

- pyrocatéchol non transformé 0,340 g - TT = 32%
- gaïacol formé 0,180 g - RT = environ 100%.

Exemple 3

Dans un tube de verre résistant à la pression, on introduit les réactifs suivants:

| | |
|---|---|
| - Acétate de sodium anhydre | 2,3 g |
| - Acide acétique | 1,9 g |
| - Eau distillée | 5 ml |
| - Méthanol | 5 ml |
| - Pyrocatéchol | 0,5 g |

On effectue l'essai dans les conditions de l'exemple 1. Le traitement du mélange réactionnel final et le dosage sont également les mêmes que dans l'exemple 1.

On trouve les résultats suivants:

| | |
|---|---|
| - TT du pyrocatéchol | 34% |
| - RT en gaïacol | 90% |

On décèle moins de 5% (RT) de diméthoxy-1,2 benzène par chromatographie.

Exemple 4

Dans un tube de verre résistant à la pression, on introduit les réactifs suivants:

| | |
|---|---|
| - Acétate de sodium anhydre | 2,3 g |
| - Acide acétique | 5,0 g |
| - Eau distillée | 5 ml |
| - Méthanol | 5 ml |
| - Pyrocatéchol | 0,5 g |

On trouve les résultats suivants:

| | |
|---|---|
| - TT du pyrocatéchol | 32% |
| - RT en gaïacol | 95% |

On décèle moins de 5% (RT) de diméthoxy-1,2 benzène par chromatographie.

Exemple 5

On effectue l'essai comme pour les exemples 1 et 2 mais avec les réactifs suivants:

| | |
|---|---|
| - Acétate de sodium anhydre | 2,3 g |
| - Acétate de méthyle | 10 ml |
| - Pyrocatéchol | 0,5015 g |

On opère dans les mêmes conditions que dans les exemples 1 et 2 mais en maintenant 4 heures à 250° et le mélange final est dosé comme précédemment après addition d'eau et traitement identique à celui de l'exemple 1.

On trouve:

| | |
|---|---|
| - TT du pyrocatéchol | 2,3% |
| - RT en gaïacol | 94,5% |

On ne décèle pas de diméthoxy-1,2 benzène par chromatographie.

Exemple 6

Dans un tube de verre résistant à la pression, on introduit les réactifs suivants:

| | |
|---|---|
| - Acétate de sodium anhydre | 2,3 g |
| - Acétate de méthyle | 5 ml |
| - Eau distillée | 5 ml |
| - Pyrocatéchol | 0,5004 g |

Le tube est scellé, puis chauffé sous agitation à 250°C et maintenu 4 heures à cette température.

Le mélange réactionnel final est traité et dosé comme dans l'exemple 1.

On trouve:

| | |
|---|---|
| - TT du pyrocatéchol | 12,35% |
| - RT en gaïacol | 100 % |

Exemple 7

Dans un tube de verre résistant à la pression, on introduit les réactifs suivants:

| | |
|---|---|
| - Acétate de sodium anhydre | 2,3 g |
| - Acide acétique | 0,38 g |
| - Eau distillée | 2 ml |
| - Méthanol | 8 ml |
| - Pyrocatéchol | 0,501 g |

On effectue l'essai dans les conditions de l'exemple 1; le traitement du mélange réactionnel final et les dosages sont également les mêmes que dans l'exemple 1.

On trouve les résultats suivants:

| | |
|---|---|
| - TT du pyrocatéchol | 75% |
| - RT en gaïacol | 80% |

On décèle environ 5% (RT) de diméthoxy-1,2 benzène par chromatographie.

Exemple 8

Dans un tube de verre résistant à la pression, on introduit les réactifs suivants:

| | |
|---|---|
| - Acétate de sodium anhydre | 2,3 g |
| - Acide acétique | 0,38 g |
| - Eau distillée | 8 ml |
| - Méthanol | 2 ml |
| - Pyrocatéchol | 0,5 g |

On effectue l'essai dans les conditions de l'exemple 1; le traitement du mélange réactionnel final et le dosage sont également les mêmes que dans l'exemple 1.

On trouve les résultats suivants:
- TT du pyrocatéchol 25%
- RT en gaïacol 96%

On décèle moins de 3% (RT) de diméthoxy-1,2 benzène par chromatographie.

Exemple 9

Dans un tube de verre résistant à la pression, on introduit les réactifs suivants:

| | |
|---|---|
| - Acétate de sodium anhydre | 2,3 g |
| - Acide acétique | 9 ml |
| - Eau distillée | 1 ml |
| - Méthanol | 5 ml |
| - Pyrocatéchol | 0,498 g |

On opère comme dans l'exemple 1.

On trouve les résultats suivants:
- TT du pyrocatéchol 16%
- RT en gaïacol 100%

Exemple 10

Dans un tube de verre résistant à la pression, on introduit les réactifs suivants:

| | |
|---|---|
| - Acétate de sodium anhydre | 2,3 g |
| - Acide acétique | 0,38 g |
| - Méthanol | 10 ml |
| - Pyrocatéchol | 0,5 g |

On opère comme dans l'exemple 1 mais l'essai est maintenu pendant 4 heures à 200°C. Le traitement du mélange réactionnel final et le dosage sont les mêmes que dans l'exemple 1 après addition d'eau.

On trouve les résultats suivants:
- TT du pyrocatéchol 24%
- RT en gaïacol 72%.

On décèle moins de 5% (RT) de diméthoxy-1,2 benzène par chromatographie.

Exemple 11

Dans un tube de verre résistant à la pression, on introduit les réactifs suivants:

| | |
|---|---|
| - Acétate de sodium anhydre | 9,18 g |
| - Acide acétique | 1,5 g |
| - Méthanol | 10 ml |
| - Pyrocatéchol | 0,5 g |

On opère comme dans l'exemple 1, mais l'essai est maintenu pendant 4 heures à 200°C. Le traitement du mélange final et le dosage sont les mêmes que dans l'exemple 1 après addition d'eau.

On trouve les résultats suivants:
- TT du pyrocatéchol 22%
- RT en gaïacol 67%

On ne décèle pas de diméthoxy-1,2 benzène par chromatographie.

Exemple 12

Dans un tube de verre résistant à la pression, on introduit les réactifs suivants:

| | |
|---|---|
| - Acétate de sodium anhydre | 2,3 g |
| - Acide acétique | 0,38 g |
| - Eau distillée | 5 ml |
| - Méthanol | 5 ml |
| - Phénol | 0,5 g |

On opère comme dans l'exemple 1.

On trouve les résultats suivants:
- TT du phénol 20%
- RT en anisole (méthoxybenzène) 75%

On ne décèle pas de présence de crésols ou de méthylanisole.

Exemple 13

Dans un tube de verre résistant à la pression, on introduit les réactifs suivants:

| | |
|---|---|
| - Acétate de sodium anhydre | 2,3 g |
| - Acide acétique | 0,38 g |
| - Eau distillée | 5 ml |
| - Méthanol | 5 ml |
| - Parachlorophénol | 0,5 g |

On opère comme dans l'exemple 1.

On obtient les résultats suviants:
- TT du parachlorophénol 37,8%
- RT en parachloroanisole 52 %

Exemple 14

Dans un tube de verre résistant à la pression, on introduit les réactifs suivants:

| | |
|---|---|
| - Acide acétique | 0,35 g |
| - Acétate de sodium anhydre | 2,3 g |
| - Eau distillée | 5 ml |
| - Ethanol | 5 ml |
| - Pyrocatéchol | 0,5 g |

On opère comme dans l'exemple 1.

On obtient avec une sélectivité (RT) d'environ 95%, le monoéthyléther de pyrocatéchol (guétol). Le taux de transformation du pyrocatéchol est voisin de 15%.

La teneur en diéthoxy-1,2 benzène est inférieure à 2%.

Exemple 15

Dans un tube de verre résistant à la pression, on introduit les réactifs suivants:

| | |
|---|---|
| - Acide acétique | 1,5 g |
| - Acétate de sodium anhydre | 9,18 g |
| - Eau distillée | 5 ml |
| - Méthanol | 5 ml |
| - Hydroquinone | 1 g. |

On opère comme dans l'exemple 1.

On obtient le monométhyléther de l'hydroquinone avec un rendement d'environ 95%, le taux de transformation de l'hydroquinone étant de 10%. On note l'absence presque totale de diméthoxy-1,4 benzène (quantité inférieure à 2%).

Exemple 16

Dans un tube de verre résistant à la pression, on introduit les réactifs suivants:

- Acide acétique 0,38 g
- Acétate de sodium anhydre 2,3 g
- Eau distillée 5 ml
- Méthanol 5 ml
- Pyrocatéchol 2 g

On opère comme dans l'exemple 1. Le traitement du mélange réactionnel final et les dosages sont effectués par chromatographie gaz/liquide et par chromatographie liquide.

On trouve les résultats suivants:
- TT du pyrocatéchol 28%
- RT en gaïacol 95%
- RT en diméthoxy-1,2 benzène 4%

Exemple 17

Dans un tube de verre résistant à la pression on introduit les réactifs suivants:

- Acide acétique 1,14 g
- Acétate de sodium anhydre 6,9 g
- Eau distillée 1,5 ml
- Méthanol 3,5 ml
- Pyrocatéchol 5 g

On opère comme dans l'exemple 1. Le traitement du mélange réactionnel final et les dosages sont effectués par chromatographie gaz/liquide et par chromatographie liquide.

On trouve les résultats suivants:
- TT du pyrocatéchol 42,5%
- RT en gaïacol 91 %
- RT en diméthoxy-1,2 benzène 9 %

Exemple 18

Dans un tube de verre résistant à la pression, on introduit les réactifs suivants:

- Acide acétique 0,19 g
- Acétate de sodium anhydre 1,15 g
- Eau distillée 5 ml
- Méthanol 5 ml
- Pyrocatéchol 2 g

On opère comme dans l'exemple 1. Le traitement du mélange réactionnel final et les dosages sont effectués par chromatographie gaz/liquide et par chromatogrpahie liquide.

On trouve les résultats suivants:
- TT du pyrocatéchol 23,5%
- RT en gaïacol 95,5%
- RT en diméthoxy-1,2 benzène 2,5%

ESSAIS COMPARATIFS:

Essai A

Dans un tube de verre résistant à la pression on introduit les réactifs suivants:

- Triéthylamine 0,5 g
- Acétate de méthyle 2,1 g
- Méthanol 3,55 g
- Pyrocatéchol 2,5 g

Le tube est scellé, puis on chauffe à 210°C sous agitation et on maintient à cette température pendant 5 h 30 mn.

Après refroidissement on dose le mélange réactionnel final par chromatographie.

On trouve les résultats suivants:
- TT du pyrocatéchol 59,5%
- RT en gaïacol 57 %
- RT en diméthoxy-1,2 benzène 20 %

On n'observe pas de sélectivité en monoéther.

Essai B

Dans un tube de verre résistant à la pression on introduit les réactifs suivants:

- Triéthylamine 1,15 g
- Méthanol 4,5 g
- Acide acétique 0,35 g
- Pyrocatéchol 2,5 g

On effectue l'essai dans les mêmes conditions que l'essai A et le mélange final est dosé comme précédemment.

On trouve les résultats suivants:
- TT du pyrocatéchol 64%
- RT en gaïacol 33%
- RT en diméthoxy-1,2 benzène 29%

On n'observe pas de sélectivité en monoéther.

Essai C

Dans un tube de verre résistant à la pression on introduit les réactifs suivants:

- Triéthylamine 1,15 g
- Méthanol 4,5 g
- Acide acétique 0,35 g
- Eau 5 ml
- Pyrocatéchol 2,5 g

On effectue l'essai dans les mêmes conditions que l'essai A, mais la durée de maintien à 210°C est de 5 heures. Le traitement consiste dans l'extraction des composés organiques par l'éther isopropylique. On dose la solution organique obtenue par chromatographie.

On trouve les résultats suivants:

- TT du pyrocatéchol 22 %
- RT en gaïacol 22,5%
- RT en diméthoxy-1,2 benzène 16,5%

On n'observe pas de sélectivité en monoéther.

**Revendications**

1. Procédé d'éthérification d'une fonction phénolique de composé de formule générale (I):

HO-AR-$(R)_n$ (I)

dans laquelle:
- Ar représente un radical aromatique constitué par un cycle benzénique ou par plusieurs cycles benzéniques orthocondensés ou ortho- et péricondensés,
- les substituants R, identiques ou différents, représentent un groupement hydroxyle, un radical alkyle linéaire ou ramifié ayant de 1 à 6 atomes de carbone, un radical alkényle linéaire ou ramifié ayant 2 à 6 atomes de carbone, un radical phényle, un radical cycloalkyle, un radical phénylalkyle dans lequel la chaîne aliphatique comporte 1 à 4 atomes de carbone, un radical cycloalkylalkyle dans lequel la chaîne aliphatique comporte de 1 à 4 atomes de carbone, un atome d'halogène, un groupement nitro, un groupement amine, un groupement aldéhyde, un groupement nitrile,
- n est un nombre de 0 à 5,
par réaction avec un agent d'éthérification choisi dans le groupe constitué par les carboxylates d'alkyle, le radical alkyle linéaire ou ramifié ayant 1 à 6 atomes de carbone, les carboxylates d'alkényle, le radical alkényle linéaire ou ramifié ayant 3 à 6 atomes de carbone et les composés susceptibles de former de tels carboxylates, caractérisé en ce que l'on opère en présence d'un sel d'acide carboxylique choisi parmi les carboxylates de métal alcalin, d'ammonium ou de métal alcalino-terreux.

2. Procédé selon la revendication 1, caractérisé en ce qu'il est appliqué à un composé de formule (I) dans laquelle:
- le radical Ar correspond au benzène, au naphtalène, à l'anthracène ou au phénanthrène
- les substituants R, identiques ou différents, représentent un groupement hydroxyle, un radical alkyle linéaire ou ramifié ayant de 1 à 4 atomes de carbone, un radical alkényle linéaire ou ramifié ayant 2 à 4 atomes de carbone, un radical phényle, un radical cyclohexyle, un radical phénylalkyle dans lequel la chaîne aliphatique comporte 1 à 3 atomes de carbone, un radical cyclohexylalkyle dans lequel la chaîne aliphatique comporte 1 à 3 atomes de carbone, un atome de chlore, un atome de brome ou un groupement nitro,
- n est un nombre entier de 0 à 3.

3. Procédé selon l'une des revendications 1 et 2, caractérisé en ce qu'il est appliqué au phénol, au naphtol-1, au naphtol-2, au méthyl-2 phénol, au méthyl-3 phénol, au méthyl-4 phénol, aux monochlorophénols, aux dichlorophénols, au monoéthylphénols, au mononitrophénols, au pyrocatéchol, au résorcinol, à l'hydroquinone, au dihydroxy-1,2 naphtalène, au dihydroxy-1,3 naphtalène, au dihydroxy-1,4 naphtalène, au dihydroxy-1,5 naphtalène, au dihydroxy-1,6 naphtalène, au dihydroxy-1,7 naphtalène, au dihydroxy-1,8 naphtalène, au dihydroxy-2,3 naphtalène, au dihydroxy-2,6 naphtalène, au dihydroxy-2,7 naphtalène.

4. Procédé selon l'une quelconque des revendications 1 à 3, caractérisé en ce que l'agent d'éthérification est choisi parmi les esters:
- des acides carboxyliques aliphatiques saturés ou insaturés monofonctionnels comportant notamment 2 à 18 atomes de carbone ou polyfonctionnels comportant notamment 3 à 18 atomes de carbone,
- des acides aromatiques mono- ou polyfonctionnels,
- des acides arylaliphatiques mono- ou polyfonctionnels,
- ou des acides cycloaliphatiques mono- ou polyfonctionnels avec des alcools tels que le méthanol, l'éthanol, le n-propanol, l'isopropanol, le butanol-1, le butanol-2, le tertiobutanol, le propène-2 ol-1, le méthyl-2 propène-2 ol-1, le butène-2 ol-1, le butène-3 ol-1, le butène-3 ol-2.

5. Procédé selon l'une quelconque des revendications 1 à 3, caractérisé en ce que l'agent d'éthérification est constitué par un mélange:
- d'un alcool tel que le méthanol, l'éthanol, le n-propanol, l'isopropanol, le butanol-1, le butanol-2, le tertiobutanol, le propène-2 ol-1, le méthyl-2 propène-2 ol-1, le butène-2 ol-1, le butène-3 ol-1, le butène-3 ol-2,
- et d'un acide carboxylique tel que:
- un acide carboxylique aliphatique saturé ou insaturé monofonctionnel comportant notamment 2 à 18 atomes de carbone ou polyfonctionnel comportant notamment 3 à 18 atomes de carbone,
- un acide aromatique mono- ou polyfonctionnel,
- un acide arylaliphatique mono- ou polyfonctionnel,
- ou un acide cycloaliphatique mono- ou polyfonctionnel.

6. Procédé selon la revendication 5, caractérisé en ce que le rapport molaire alcool/acide carboxylique est compris entre 0,02 et 50 et de préférence entre 0,1 et 40.

7. Procédé selon l'une des revendications 4 à 6, caractérisé en ce que l'acide carboxylique entrant dans la composition de l'agent d'éthérification est un acide aliphatique saturé mono- ou difonctionnel ayant de 2 à 6 atomes de carbone, l'acide benzoïque ou un des acides ortho-, méta- et téréphtaliques.

8. Procédé selon l'une quelconque des revendications 1 à 7, caractérisé en ce que le sel d'acide carboxylique utilisé est un carboxylate de métal alcalin.

9. Procédé selon l'une quelconque des revendications 1 à 8, caractérisé en ce que le sel d'acide carboxylique utilisé est un sel d'un acide aliphatique saturé mono- ou difonctionnel ayant

2 à 6 atomes de carbone, de l'acide benzoïque ou de l'un des acides ortho-, méta- et téréphtaliques.

10. Procédé selon l'une quelconque des revendications 1 à 9, caractérisé en ce que l'on opère en présence d'eau.

11. Procédé selon la revendication 10, caractérisé en ce que l'eau représente de 1% à 95% en volume du milieu réactionnel liquide et de préférence de 20% à 80%.

12. Procédé selon l'une des revendications 1 à 3 et 5 et 6, caractérisé en ce que l'on utilise un diacide carboxylique dont une fonction acide est libre et l'autre est salifiée.

13. Procédé selon l'une quelconque des revendications 1 à 12, caractérisé en ce que l'on opère à une température située entre 150°C et 350°C, et de préférence entre 220°C et 300°C.

14. Procédé selon l'une quelconque des revendications 1 à 13, caractérisé en ce que l'on utilise un rapport molaire ester ou alcool entrant dans la composition de l'agent d'éthérification/composé phénolique égal ou supérieur à 0,5, et de préférence égal ou supérieur à 1.

15. Procédé selon l'une quelconque des revendications 1 à 14, caractérisé en ce que les éthers formés se trouvant dans la masse réactionnelle finale sont extraits par le cyclohexane.

**Patentansprüche**

1. Verfahren zur Verätherung einer phenolischen Funktion einer Verbindung der allgemeinen Formel I

$$HO-Ar-(R)_n \qquad I$$

worin:
- Ar einen aromatischen Rest bedeutet, bestehend aus einem Benzolring oder aus mehreren orthokondensierten oder ortho- und perikondensierten Benzolringen,
- die Substituenten R, welche identisch oder verschieden sind, eine Hydroxylgruppe, einen geraden oder verzweigten Alkylrest mit 1 bis 6 Kohlenstoffatomen, einen geraden oder verzweigten Alkenylrest mit 2 bis 6 Kohlenstoffatomen, einen Phenylrest, einen Cycloalkylrest, einen Phenylalkylrest, worin die aliphatische Kette 1 bis 4 Kohlenstoffatome trägt, einen Cycloalkylalkylrest, worin die aliphatische Kette 1 bis 4 Kohlenstoffatome hat, ein Halogenatom, eine Nitrogruppe, eine Aminogruppe, eine Aldehydgruppe oder eine Nitrilgruppe bedeuten,
- n eine Zahl von Null bis 5 ist,

durch Reaktion mit einem Verätherungsmittel, ausgewählt aus der Gruppe bestehend aus den Carbonsäurealkylestern, wobei der Alkylrest linear oder verzweigt mit 1 bis 6 Kohlenstoffatomen ist, den Alkenylcarbonsäureestern, wobei der gerade oder verzweigte Alkenylrest 3 bis 6 Kohlenstoffatome hat und den Verbindungen, welche derartige Carbonsäureester zu bilden vermögen,
dadurch gekennzeichnet, dass man in Gegnewart eines Carbonsäuresalzes, ausgewählt aus den Alkalimetallcarboxylaten, Ammoniumcarboxylaten oder Erdalkalimetallcarboxylaten arbeitet.

2. Verfahren gemäss Anspruch 1, dadurch gekennzeichnet, dass es auf eine Verbindung der Formel I angewendet wird, worin:
- der Rest Ar dem Benzol, Naphthalin, Anthracen oder Phenanthren entspricht,
- die Substituenten R, welche identisch oder verschieden sind, eine Hydroxylgruppe, einen geraden oder verzweigten Alkylrest mit 1 bis 4 Kohlenstoffatomen, einen geraden oder verzweigten Alkenylrest mit 2 bis 4 Kohlenstoffatomen, einen Phenylrest, einen Cyclohexylrest, einen Phenylalkylrest, worin die aliphatische Kette 1 bis 3 Kohlenstoffatome trägt, einen Cyclohexylalkylrest, worin die aliphatische Kette 1 bis 3 Kohlenstoffatome trägt, ein Chloratom, ein Bromatom oder eine Nitrogruppe bedeuten,
- n eine ganze Zahl von Null bis 3 ist.

3. Verfahren gemäss einem der Ansprüche 1 und 2, dadurch gekennzeichent, dass es angewandt wird auf Phenol, 1-Naphthol, 2-Naphthol, auf 2-Methylphenol, auf 3-Methylphenol, auf 4-Methylphenol, auf die Monochlorphenole, auf die Dichlorphenole, auf Monoäthylphenole, auf Mononitrophenole, auf Brenzcatechin, auf Resorcin, auf Hydrochinon, auf 1,2-Dihydroxynaphthalin, auf 1,3-Dihydroxynaphthalin, auf 1,4-Dihydroxynaphthalin, auf 1,5-Dihydroxynaphthalin, auf 1,6-Dihydroxynaphthalin, auf 1,7-Dihydroxynaphthalin, auf 1,8-Dihydroxynaphthalin, auf 2,3-Dihydroxynaphthalin, auf 2,6-Dihydroxynaphthalin, auf 2,7-Dihydroxynaphthalin.

4. Verfahren gemäss einem der Ansprüche 1 bis 3, dadurch gekennzeichnet, dass das Verätherungsmittel ausgewählt wird unter den Estern:
- der gesättigten oder ungesättigten monofunktionellen aliphatischen Carbonsäuren mit insbesondere 2 bis 18 Kohlenstoffatomen oder polyfunktionellen mit insbesondere 3 bis 18 Kohlenstoffatomen,
- der mono- oder polyfunktionellen aromatischen Säuren,
- der mono- oder polyfunktionellen arylaliphatischen Säuren,
- oder der mono- oder polyfunktionellen cycloaliphatischen Säuren mit Alkoholen wie Methanol, Äthanol, n-Propanol, Isopropanol, 1-Butanol, 2-Butanol, tert-Butanol, Prop-2-en-1-ol, 2-Methylprop-2-en-1-ol, But-2-en-1-ol, But-3-en-1-ol, But-3-en-2-ol.

5. Verfahren nach einem der Ansprüche 1 bis 3, dadurch gekennzeichnet, dass das Verätherungsmittel besteht aus einem Gemisch:
- eines Alkohols wie Methanol, Äthanol, n-Propanol, Isopropanol, Butanol-(1), Butanol-(2), t-Butanol, Propen-(2)-ol-(1), 2-Methylpropen-(2)-ol-(1), Buten-(2)-ol-(1), Buten-(3)-ol-(1), Buten-(3)-ol-(2)
- und einer Carbonsäure wie
- eine monofunktionelle gesättigte oder ungesättigte aliphatische Carbonsärue mit insbesondere 2 bis 18 Kohlenstoffatomen oder polyfunktionelle mit insbesondere 3 bis 18 Kohlenstoffatomen,
- eine mono- oder polyfunktionelle aromatische Säure,
- eine mono- oder polyfunktionelle arylaliphatische Säure,
- oder eine mono- oder polyfunktionelle cycloaliphatische Säure.

6. Verfahren gemäss Anspruch 5, dadurch gekennzeichnet, dass das Molverhältnis Alkohol/Carbonsäure zwischen 0,02 und 50 und vorzugsweise zwischen 0,1 und 40 liegt.

7. Verfahren gemäss einem der Ansprüche 4 bis 6, dadurch gekennzeichnet, dass die Carbonsäure, welche in die Zusammensetzung des Verätherungsmittels eintritt, eine mono- oder difunktionelle gesättigte aliphatische Säure mit 2 bis 6 Kohlenstoffatomen, Dibenzoesäure oder eine der Ortho-, Meta- oder Terephthalsäuren ist.

8. Verfahren gemäss einem der Ansprüche 1 bis 7, dadurch gekennzeichnet, dass das verwendete Salz der Carbonsäure ein Alkalimetallcarboxylat ist.

9. Verfahren gemäss einem der Ansprüche 1 bis 8, dadurch gekennzeichnet, dass das verwendete Carbonsäuresalz ein Salz einer mono- oder difunktionellen gesättigten aliphatischen Säure mit 2 bis 6 Kohlenstoffatomen, der Benzoesäure oder einer der Ortho-, Meta- und Terepthalsäuren ist.

10. Verfahren gemäss einem der Ansprüche 1 bis 9, dadurch gekennzeichnet, dass man in Gegenwart von Wasser arbeitet.

11. Verfahren gemäss Anspruch 10, dadurch gekennzeichnet, dass das Wasser 1 bis 95 Vol-% des flüssigen Reaktionsmilieus und vorzugsweise 20 bis 80% ausmacht.

12. Verfahren gemäss einem der Ansprüche 1 bis 3 und 5 und 6, dadurch gekennzeichnet, dass man eine Dicarbonsäure verwendet, deren eine Säurefunktion frei und die andere in Salzform ist.

13. Verfahren gemäss einem der Ansprüche 1 bis 12, dadurch gekennzeichnet, dass man bei einer Temperatur zwischen 150 un 350°C, vorzugsweise zwischen 220 und 300°C arbeitet.

14. Verfahren gemäss einem der Ansprüche 1 bis 13, dadurch gekennzeichnet, dass man ein Molverhältnis Ester oder Alkohol, der in die Zusammensetzung des Verätherungsmittels eintritt/phenolische Komponente gleich oder grösser 0,5 und vorzugsweise gleich oder grösser 1 verwendet.

15. Verfahren gemäss einem der Ansprüche 1 bis 14, dadurch gekennzeichnet, dass die gebildeten Äther, welche sich in der endgültigen Reaktionsmasse befinden, mit Cyclohexan extrahiert werden.

## Claims

1. Process for the etherification of a phenol group in a compound of the general formula (I):

$$HO\text{-}Ar\text{-}(R)_n \qquad (I)$$

in which:
- Ar represents an aromatic radical consisting of a benzene ring or of several ortho-fused or ortho- and perifused benzene rings,
- the substituents R, which are identical or different, represent a hydroxyl group, a linear or branched alkyl radical having from 1 to 6 carbon atoms, a linear or branched alkenyl radical having 2 to 6 carbon atoms, a phenyl radical, a cycloalkyl radical, a phenylalkyl radical in which the aliphatic chain contains 1 to 4 carbon atoms, a cycloalkyl-alkyl radical in which the aliphatic chain contains from 1 to 4 carbon atoms, a halogen atom, a nitro group, an amine group, an aldehyde group or a nitrile group, and
- n is a number from 0 to 5,

by reaction with an etherifying agent chosen from the group comprising alkyl carboxylates, the linear or branched alkyl radical having 1 to 6 carbon atoms, alkenyl carboxylates, the linear or branched alkenyl radical having 3 to 6 carbon atoms, and compounds capable of forming such carboxylates, characterised in that the reaction is carried out in the presence of a carboxylic acid salt chosen from amongst alkali metal, ammonium or alkaline earth metal carboxylates.

2. Process according to Claim 1, characterised in that it is applied to a compound of the formula (I) in which:
- the radical Ar corresponds to benzene, naphthalene, anthracene or phenanthrene,
- the substituents R, which are identical or different, represent a hydroxyl group, a linear or branched alkyl radical having from 1 to 4 carbon atoms, a linear or branched alkenyl radical having 2 to 4 carbon atoms, a phenyl radical, a cyclohexyl radical, a phenylalkyl radical in which the aliphatic chain contains 1 to 3 carbon atoms, a cyclohexyl-alkyl radical in which the aliphatic chain contains 1 to 3 carbon atoms, a chlorine atom, a bromine atom or a nitro group, and
- n is an integer from 0 to 3.

3. Process according to one of Claims 1 and 2, characterised in that it is applied to phenol, 1-naphthol, 2-naphthol, 2-methylphenol, 3-methylphenol, 4-methylphenol, monochlorophenols, dichlorophenols, monoethylphenols, mononitrophenols, pyrocatechol, resorcinol, hydroquinone, 1,2-dihydroxynaphthalene, 1,3-dihydroxynaphthalene, 1,4-dihydroxynaphthalene, 1,5-dihydroxynaphthalene, 1,6-dihydroxynaphthalene, 1,7-dihydroxynaphthalene, 1,8-dihydroxynaphthalene, 2,3-dihydroxynaphthalene, 2,6-dihydroxynaphthalene, 2,7-dihydroxynaphthalene.

4. Process according to any one of Claims 1 to 3, characterised in that the etherifying agent is chosen from amongst the esters of:
- monofunctional, saturated or unsaturated aliphatic carboxylic acids containing in particular 2 to 18 carbon atoms, or polyfunctional, saturated or unsaturated aliphatic carboxylic acids containing in particular 3 to 18 carbon atoms,
- monofunctional or polyfunctional aromatic acids,
- monofunctional or polyfunctional arylaliphatic acids, or

- monofunctional or polyfunctional cycloaliphatic acids
with alcohols such as methanol, ethanol, n-propanol, isopropanol, butan-1-ol, butan-2-ol, tert.-butanol, prop-2-en-1-ol, 2-methylprop-2-en-1-ol, but-2-en-1-ol, but-3-en-1-ol or but-3-en-2-ol.

5. Process according to any one of Claims 1 to 3, characterised in that the etherifying agent consists of a mixture of:
- an alcohol such as methanol, ethanol, n-propanol, isopropanol, butan-1-ol, butan-2-ol, tert.-butanol, prop-2-en-1-ol, 2-methylprop-2-en-1-ol, but-2-en-1-ol, but-3-en-1-ol or but-3-en-2-ol, and
- a carboxylic acid such as:
- a monofunctional, saturated or unsaturated aliphatic carboxylic acid containing in particular 2 to 18 carbon atoms, or a polyfunctional, saturated or unsaturated aliphatic carboxylic acid containing in particular 3 to 18 carbon atoms,
- a monofunctional or polyfunctional aromatic acid,
- a monofunctional or polyfunctional arylaliphatic acid, or
- a monofunctional or polyfunctional cycloaliphatic acid.

6. Process according to Claim 5, characterised in that the molar ratio of alcohol/carboxylic acid is between 0.02 and 50 and preferably between 0.1 and 40.

7. Process according to one of Claims 4 to 6, characterised in that the carboxylic acid forming part of the composition of the etherifying agent is a monofunctional or difunctional, saturated aliphatic acid having from 2 to 6 carbon atoms, benzoic acid or orthophthalic, metaphthalic or terephthalic acid.

8. Process according to any one of Claims 1 to 7, characterised in that the carboxlylic acid salt used is an alkali metal carboxylate.

9. Process according to any one of Claims 1 to 8, characterised in that the carboxylic acid salt used is a salt of a monofunctional or difunctional, saturated aliphatic acid having 2 to 6 carbon atoms, of benzoic acid or of orthophthalic, metaphthalic or terephthalic acid.

10. Process according to any one of Claims 1 to 9, characterised in that the reaction is carried out in the presence of water.

11. Process according to Claim 10, characterised in that the water represents from 1% to 95% by volume of the liquid reaction medium, and preferably from 20% to 80%.

12. Process according to one of Claims 1 to 3 and 5 and 6, characterised in that a dicarboxylic acid is used in which one acid group is free and the other is salified.

13. Process according to any one of Claims 1 to 12, characterised in that the reaction is carried out at a temperature of between 150°C and 350°C and preferably of between 220°C and 300°C.

14. Process according to any of one of Claims 1 to 13, characterised in that a molar ratio of ester or alcohol forming part of the composition of the etherifying agent/phenolic compound is used which is equal to or greater than 0.5 and preferably equal to or greater than 1.

15. Process according to any one of Claims 1 to 14, characterised in that the ethers formed and present in the final reaction mixture are extracted with cyclohexane.